# EUROPÄISCHE PATENTSCHRIFT

(11) **EP 3 235 467 B1**
(45) Veröffentlichungstag und Bekanntmachung des Hinweises auf die Patenterteilung: **31.03.2021**
(21) Anmeldenummer: 16166705.0
(22) Anmeldetag: 22.04.2016
(51) Int. Cl.: A61C 13/00, A61C 13/09

(54) **VERFAHREN ZUR HERSTELLUNG EINER DENTALRESTAURATION**
METHOD FOR FABRICATING A DENTAL RESTORATION
MÉTHODE DE FABRICATION D'UNE RESTAURATION DENTAIRE

(43) Veröffentlichungstag der Anmeldung: 25.10.2017
(73) Patentinhaber: Ivoclar Vivadent AG, 9494 Schaan (LI)
(72) Erfinder: Krolikowski, Sebastian, 8853 Lachen (CH); Rampf, Markus, 7212 Seewis-Dorf (CH)
(74) Vertreter: Uexküll & Stolberg

(56) Entgegenhaltungen:
- EP-A1- 1 132 056
- EP-B1- 2 065 012
- WO-A1-02/09612
- JP-A- H11 216 148
- US-A1- 2009 026 643
- US-A1- 2015 335 407

## Beschreibung

Die vorliegende Erfindung betrifft ein Verfahren zur Herstellung einer Dentalrestauration, bei dem ein Rohling aus Keramik, insbesondere Glaskeramik, oder Glas bereitgestellt wird, der eine vorgegebene Ortsabhängigkeit der Materialeigenschaft Farbe und/oder Opazität in seinem Volumen aufweist, und der Rohling in einen Pressraum einer Pressmuffel gepresst und dadurch Material des Rohlings durch eine von dem Pressraum ausgehende Kanalstruktur in einen der Dentalrestauration entsprechenden Formhohlraum gepresst wird, wobei die Kanalstruktur und die Ortsabhängigkeit von Farbe und/oder Opazität im Rohling aufeinander abgestimmt sind, so dass sich eine aus einer Mehrzahl von möglichen Verteilungen von Farbe und/oder Opazität im keramischen Material der im Formhohlraum gebildeten Dentalrestauration ergibt.

Bereits seit einiger Zeit sind Verfahren bekannt, mit denen Dentalrestaurationen aus keramischem Material "automatisiert" mit CAD-CAM-Systemen hergestellt werden können. Nach einer digitalen Aufnahme (Scannen im Mund des Patienten oder an einem abgeformten Modell) kann die Dentalrestauration auf Basis der Scandaten mit einer Fräsmaschine aus vollem Material gefräst bzw. geschliffen werden. Diese abtragenden Herstellungsverfahren haben allerdings auch Nachteile, z.B. dass ein großer Teil der eingesetzten, wertvollen Glas- oder Glaskeramikmaterialien verloren geht. Darüber hinaus sind die eingesetzten Maschinen teuer und wartungsintensiv.

Neben den abtragenden Verfahren werden auch sogenannte aufbauende Verfahren angewendet, die auch unter den Begriffen "Rapid prototyping" oder "generative Fertigung" bekannt sind. Beispiele dafür sind Stereolithografie-, 3-D-Pulverdruck- und 3-D-Tintenstrahldruckverfahren, wobei letzteres auch als Ink Jet Printing bezeichnet wird. Einige aufbauende Verfahren beruhen auf dem schichtweisen Aufbau eines dreidimensionalen Formkörpers, wobei die zweidimensionalen Schichten mit jeweils vorgegebener Kontur aufeinander aufgebaut werden.

Daneben sind auch Press- oder Einpressverfahren zur Herstellung von Dentalrestaurationen bekannt. Dabei wird ein Modell der herzustellenden Dentalrestauration, das aus einem vollständig ausbrennbaren Material besteht, an einem eine spätere Kanalstruktur bildenden Negativform der Kanalstruktur (auch als Anstiftsystem bezeichnet) aus ebensolchem Material innerhalb einer Pressmuffel angebracht, wobei das vom Formhohlraum weg weisende Ende der Negativform an einem Vorsprung am Boden der Pressmuffel befestigt wird. Anschließend wird die Pressmuffel mit einer Einbettungsmasse gefüllt, so dass das Modell der Dentalrestauration und die Negativform der Kanalstruktur vollständig von der Einbettungsmasse umgeben sind. Die Einbettungsmasse wird zu einer feuerfesten Pressform ausgehärtet und das Modell und die Negativform der Kanalstruktur werden ausgebrannt, um einen dem Modell der Dentalrestauration entsprechenden (komplementären) Formhohlraum und die zu ihm führende offene Kanalstruktur in der ausgehärteten Pressform zu erzeugen. Der Boden der Pressmuffel wird abgenommen, wonach in der Pressform an der Stelle des Vorsprungs ein Aufnahme- oder Pressraum zurückbleibt, von dem die Kanalstruktur zu dem Formhohlraum ausgeht. In den Pressraum wird ein zum Pressraum im wesentlich komplementär geformter Rohling z.B. aus Keramik eingeführt und unter Druck- und meist Hitzeanwendung gepresst, so dass keramisches Material des Rohlings durch die Kanalstruktur in den Formhohlraum gepresst wird, um den Formhohlraum vollständig auszufüllen und so die Dentalrestauration in der gewünschten Form herzustellen. Ein derartiges Verfahren, das nach dem beschriebenen Prinzip der verlorenen Form arbeitet, ist z.B. aus EP 2 952 154 A1 bekannt.

Aus EP 2 065 012 B1 ist ein Verfahren nach dem Oberbegriff von Patentanspruch 1 bekannt, das ebenfalls nach dem Prinzip der verlorenen Form arbeitet. Bei diesem Verfahren werden keramische Rohlinge eingesetzt, deren Volumen in zwei oder mehr Volumenbereiche unterteilt sind, die sich hinsichtlich der Materialeigenschaft Farbe und/oder Opazität unterscheiden. Die Materialeigenschaft Farbe und/oder Opazität muss sich beim Übergang von einem in einen anderen Volumenbereich nicht sprunghaft ändern, sondern kann einen fließenden Übergang bilden. Ein Beispiel ist ein zylindrischer Rohling, der entlang einer die Zylinderlängsachse enthaltenden Ebene in Hälften unterteilt ist, wobei sich das Material in der einen Hälfte hinsichtlich seiner Farbe von dem in der anderen Hälfte unterscheidet. In der von dem Pressraum, in den der Rohling eingeführt wird, zu dem Formhohlraum führenden Kanalstruktur sind ein oder mehrere Kanäle mit solchen Formgestaltungen (abzweigende Blindkanäle oder Erweiterungen oder Hohlräume) ausgebildet, dass sich für die resultierenden Fließwege unterschiedlichen Fließzeiten von dem Presshohlraum zu dem Formhohlraum ergeben. Dabei sind die Kanalstruktur und die Ortsabhängigkeit der Materialeigenschaft Farbe und/oder Opazität in dem Rohling so aufeinander abgestimmt, dass sich eine aus einer Mehrzahl von möglichen Farb- oder Opazitätsverteilungen in dem Material im Formhohlraum ergibt, wenn dieser mit Material aus dem Rohling gefüllt ist. Für die bekannten Pressverfahren wird ein Sortiment an Standard-Rohlingen bereitgestellt, die über eine Anzahl von vorgegebenen, verschiedenen Verteilungen von Farbe bzw. Opazität in ihrem Volumen verfügen, so dass sich in der Dentalrestauration je nach Auswahl des Rohlings aus dem Sortiment eine aus einer Mehrzahl von möglichen Farb- oder Opazitätsverteilungen ergibt.

Mit derartigen Pressverfahren ist es möglich, Dentalrestaurationen mit einem näherungsweise an einen gewünschten Farb- oder Opazitätsverlauf angepassten Verlauf in einem Pressschritt herzustellen. Der Kunde ist bei diesen Verfahren jedoch auf ein Sortiment von vorgefertigten Standard-Rohlingen mit jeweils definiertem ortsabhängigen Opazitäts- und Farbverlauf angewiesen, aus dem dann der am besten zu dem gewünschten Verlauf in der Dentalrestauration passende auszuwählen ist. Durch individuelle Gestaltung der Geometrie der Kanalstruktur kann der Verlauf der Fließvorgänge zwar in gewissem Umfang an die gewünschte Verteilung der Materialeigenschaften (Farbe und/oder Opazität) in eine gewünschte Richtung angepasst werden, eine genaue Anpassung an eine gewünschte Verteilung von Farbe und/oder Opazität ist aber nicht möglich. Neben der Begrenztheit des Sortiments von Standard-Rohlingen ist es dem Kunden auch nicht möglich, eventuelle feine Strukturmerkmale, z.B. Schmelzrisse, Mamelons, Schmelzflecken, etc. eines menschlichen Zahns in die herzustellende Dentalrestauration abzubilden. Soll eine Dentalrestauration insoweit individuell gestaltet werden, sind zusätzliche zeitaufwendige Nachbearbeitungsschritte des Zahntechnikers notwendig, wie z.B. das sogenannte Cut-Back, bei dem Oberflächenbereiche der Dentalrestauration wieder abgetragen werden und in den abgetragenen Bereichen neue Schichten mit abweichenden optischen Eigenschaften gebildet werden.

Den Nachteilen der fehlenden Individualität der verpressten Dentalrestaurationen wird in der Regel durch nachfolgende Schritte wie Bemalen oder das zuvor genannte Cut-Back und nachfolgendem Aufbau von Schichten mit keramischer Schichtmasse begegnet. Neben dem sehr hohen Zeitaufwand hat insbesondere das Aufbringen von Schichten einen entscheidenden Nachteil. Während der Pressrohling aus hochfester Glaskeramik (z.B. Lithium-Disilikat) mit einer Festigkeit von > 360 MPa besteht, bestehen konventionelle Schichtmassen für Lithium-Disilikat aus Fluor-Apatit oder Feldspat-Glaskeramiken mit einer Festigkeit von etwa 100 MPa. Aufgrund der geringeren Festigkeit der aus solchen Schichtmassen aufgebrachten Schichten ist die Festigkeit für beanspruchte Bereiche wie Inzisalkanten deutlich reduziert, was zu einem frühzeitigen Versagen der Dentalrestauration führen kann.

Es ist Aufgabe der vorliegenden Erfindung, ein Verfahren zur Herstellung von Dentalrestaurationen anzugeben, mit dem optische Strukturmerkmale in der herzustellenden Dentalrestauration besser und einfacher individuell gestaltbar sind.

Zur Lösung dieser Aufgabe dient das Verfahren mit den Merkmalen des Patentanspruchs 1. Vorteilhafte Ausführungsformen des Verfahrens sind in den Unteransprüchen aufgeführt.

Gemäß der vorliegenden Erfindung ist vorgesehen, dass aus einer in der herzustellenden Dentalrestauration gewünschten Verteilung von Farbe und/oder Opazität und gegebenenfalls von optischen Strukturmerkmalen (Schmelzrisse, Mamelons, Schmelzflecken, etc.) unter Berücksichtigung der Fließwege in der Kanalstruktur die zugrunde liegende Ortsabhängigkeit von Farbe und/oder Opazität im Rohling bestimmt wird, aus der die gewünschte Verteilung nach Pressen des Rohlings zu der Dentalrestauration resultiert. Die individuell benötigte Ortsabhängigkeit von Farbe und/oder Opazität in einem Rohling wird bestimmt, indem
(i) entweder die in der herzustellenden Dentalrestauration gewünschte Verteilung von Farbe und/oder Opazität und von optischen Strukturmerkmalen einer zeitlich rückwärts verlaufenden Simulation der Fließvorgänge beim Pressen des Materials durch die Kanalstruktur in den Formhohlraum unterzogen wird, um so genau diejenige Ortsabhängigkeit von Farbe und/oder Opazität im Rohling zu erhalten, die nach Pressen des Materials in den Formhohlraum dort die gewünschte Verteilung von Farbe und/oder Opazität ergibt,
(ii) oder eine vorgegebene Ortsabhängigkeit von Farbe und/oder Opazität und/oder von optischen Strukturmerkmalen im Rohling einer zeitlich vorwärts verlaufenden Simulation der Fließvorgänge beim Pressen des Materials durch die Kanalstruktur in den Formhohlraum unterzogen wird, um so die jeweils resultierende Ortsabhängigkeit von Farbe und/oder Opazität und/oder Strukturmerkmalen im Formhohlraum zu erhalten, und die genannte vorgegebene Ortsabhängigkeit im Rohling variiert und die zeitlich vorwärts verlaufende Simulation wiederholt wird, bis die resultierende Ortsabhängigkeit von Farbe und/oder Opazität und/oder Strukturmerkmalen im Formhohlraum mit der gewünschten Verteilung von Farbe und/oder Opazität und/oder von Strukturmerkmalen in der Dentalrestauration übereinstimmt.

Die Simulation der Fließvorgänge beim Fließen des Materials, wenn dieses durch die Kanalstruktur und in den Formhohlraum gepresst wird, ist mit hoher Genauigkeit möglich und kann in eine zeitliche rückwärts gerichtete Simulation überführt werden, die dann eine Abbildung der gewünschten Ortsabhängigkeit von Farbe und/oder Opazität und von Strukturmerkmalen in der fertigen Dentalrestauration zu der diese beim Pressvorgang erzeugenden Ortsabhängigkeit im Rohling für das Pressverfahren ausführt. Die Simulation des Füllprozesses durch die Kanalstruktur kann über fluidmechanische Berechnungen erfolgen, zum Beispiel durch einen statistischen Ansatz, auf Partikel basierend oder auf einem Gitternetzmodell (Finite-Elemente-Methode). Die Durchführung solcher Fließsimulationen sind für viele Materialien bekannt und verfügbar und zum Teil Bestand kommerzieller Software.

Dann wird dieser Rohling bereitgestellt, indem er in einem aufbauenden Verfahren mit der bestimmten Ortsabhängigkeit von Farbe und/oder Opazität individuell für die in diesem Fall herzustellende Dentalrestauration aufgebaut wird.

Erfindungsgemäß wird also für eine durch ein Pressverfahren aus einem Rohling herzustellende Dentalrestauration ein individuell angepasster Rohling durch ein aufbauendes, generatives Verfahren hergestellt, dessen Farb- und Opazitätsverteilung gerade so abgestimmt ist, dass sich nach dem Pressen in den Formhohlraum dort genau die gewünschte Ortsabhängigkeit von Farbe und/oder Opazität und die gegebenenfalls gewünschten Strukturmerkmale ergeben. Neben dem generellen Farb- und Opazitätsverlauf können durch den schichtweisen Aufbau des Rohlings zusätzliche Strukturierungen (z.B. Mamelons, Schmelzrisse, Schmelzflecken, etc.) eingebracht werden, die durch die hohe Ortstreue beim Pressvorgang in der hergestellten Dentalrestauration genau positioniert werden.

Anders als in der oben genannten EP 2 065 012 B1 muss bei dem Verfahren der vorliegenden Erfindung keine individuell angepasste Kanalstruktur verwendet werden, da auch mit einer Standard-Kanalstruktur durch vollständige Individualisierung der Verteilung der Materialeigenschaften im Rohling jede gewünschte Verteilung der Materialeigenschaften (Farbe und/oder Opazität und/oder optische Strukturmerkmale) in der verpressten Dentalrestauration herstellbar ist.

Soweit in der vorliegenden Anmeldung von einem Rohling aus Keramik, insbesondere Glaskeramik, oder aus Glas die Rede ist, soll dies nicht ausschließen, dass der Rohling zumindest vor dem Pressvorgang noch Bindemittel enthalten kann. Desweiteren soll die Formulierung aus Keramik, insbesondere Glaskeramik, oder Glas natürlich nicht ausschließen, dass weitere Bestandteile wie Farben in dem Rohling enthalten sein können. Der Rohling muss dabei auch nicht die Endeigenschaften der verpressten Restauration besitzen, d.h. er muss nicht dicht gesintert sein, sondern kann porös sein; er kann auch andere Festigkeiten und/oder andere kristalline Phasen aufweisen. Andererseits ist es auch möglich, dass der Rohling schon gesintert bzw. vorgesintert ist.

In einer bevorzugten Ausführungsform wird der Rohling vor oder beim Pressen erhitzt. Einerseits ist das Erhitzen (Heißpressen) ohnehin bevorzugt, um die Fließfähigkeit des Materials beim Pressvorgang zu verbessern. Bei Rohlingen, die mit aufbauenden Verfahren unter Einsatz von Bindemitteln hergestellt worden sind, dient das Erhitzen gleichzeitig dazu, den Rohling zu entbindern und vorzusintern. Bindemittel kommen zum Beispiel beim 3-D-Pulverdrucken und beim 3-D-Tintenstrahldrucken zum Einsatz, wobei die Farbe und Opazität im Rohling durch Einfärbung des Bindemittels oder durch separat gedruckte Farbe selektiv gesteuert wird. Nach Entfernung des Bindemittels bleibt der gewünschte Farb- und Opazitätsverlauf erhalten. Auf diese Weise ist es möglich, den Rohling direkt nach seinem Aufbau ohne weitere Zwischenschritte direkt in den Pressraum der Pressmuffel einzusetzen, dabei zu erhitzen und die Dentalrestauration durch Pressen in den Formhohlraum herzustellen. Weitere Zwischenschritte wie eine gesonderte Entbinderung oder Vorsinterung sind nicht notwendig.

Durch den Pressvorgang erhält das Material der Dentalrestauration (z.B. Lithium-Disilikat) eine sehr hohe Passgenauigkeit und die gleichen mechanischen Eigenschaften wie herkömmlich verpresstes Lithium-Disilikat. Der Vorteil der Herstellung eines Rohlings über ein aufbauendes Verfahren ist die vollständige Individualisierbarkeit des Rohlings und Anpassung an die gewünschten Farb- und Opazitätsverläufe in der hergestellten Dentalrestauration. Der Aufbauvorgang des Rohlings, z.B. durch 3-D-Pulverdruck oder durch 3-D-Tintenstrahldruck, ist sehr schnell und einfach, da die Geometrie des Rohlings einfach sein kann; es können zum Beispiel zylindrische oder kubische Rohlinge verwendet werden, die sehr einfach und schnell aufgebaut werden können. Dabei können beim 3-D-Pulverdruck oder beim 3-D-Tintenstrahldruck individuell gefärbte Bindemittel verdruckt werden. Aufgrund der einfachen Geometrie der Rohlinge können die Aufbauvorgänge mit einem 3-D-Pulverschichtdruck oder einem 3-D-Tintenstrahldruck sehr schnell und ohne hohe Anforderungen an die Ortsauflösung durchgeführt werden, was gleichzeitig den gerätetechnischen Aufwand für die aufbauende Herstellung der Rohlinge gering hält.

Dabei ist eine individuelle Gestaltung des Rohlings möglich, z.B. eine gewünschte Schichtfolge von transluzenteren/opakeren Schichten, wobei durch ortsaufgelöste Druckvorgänge Farbe und/oder Opazität auch über die Fläche einer Schicht variieren können. Durch die hohe Schicht- und Ortstreue beim Pressvorgang ist es auch möglich Strukturen wie Mamelons, Schmelzrisse, Schmelzfehler im Rohling ortsselektiv zu definieren, so dass die Struktur in der Dentalrestauration an der gewünschten Stelle positioniert wird. Der Rohling ist dabei räumlich in X-, Y- und Z-Richtung in diesen 3 Dimensionen mit einer gewünschten räumlichen Verteilung von Farbe und/oder Opazität und/oder Strukturmerkmalen gestaltet. Dabei ist insbesondere die Z-Richtung, also die Rohlings-Längsachse, in deren Richtung der Rohling gepresst wird, wichtig für den Opazitätsverlauf oder einen Schmelzriss als Strukturmerkmal.

Durch das Verpressen des schichtweise aufgebauten Rohlings bekommt das Material die Endgeometrie der Dentalrestauration (mit sehr hoher Genauigkeit) und seine Endeigenschaften wie bei herkömmlichen Pressprozessen.

In einer bevorzugten Ausführungsform wird für den Aufbau des Rohlings Glas mit Keimen verwendet.

In einer alternativen Ausführungsform wird für den Aufbau des Rohlings eine Glaskeramik mit der Hauptkristallphase Lithium-Metasilicat, Lithium-Disilicat oder SiO₂-Phasen oder Zwischenstufen davon verwendet wird. Derartige Materialien sind zum Beispiel in WO 2015/173 394 A1 beschrieben.

Zum schichtweisen Aufbau des Rohling sind 3-D-Pulverdruck- und 3-D-Tintenstrahldruckverfahren besonders geeignet. Bei 3-D-Pulverdruckverfahren wird ein Drucker mit zwei Wannen verwendet, die jeweils einen mit einem Druckkolben Wannenboden aufweisen. Eine der Wannen ist mit dem Pulver für das Bauverfahren gefüllt, die zweite Wanne daneben verfügt ebenfalls über einen höhenverstellbaren Wannenboden. Der Druck des Objektes beginnt durch Absenken des Wannenbodens der zweiten Wanne um eine Schichtdicke, wonach mittels eines Auftragsarmes, der Pulver aus der ersten Wanne zu der zweiten Wanne transportiert, die erste Pulverschicht auf den Wannenboden der zweiten Wanne aufgetragen wird. Diese wird nun mit einem Drucker mit einem selektiv über Pigmente, Partikel oder Binder einfärbbaren Tintenstrahl in der für die Schicht gewünschten Formen gebunden und durch selektive Steuerung der Farbe des Tintenstrahls ortsabhängig mit Farbe versehen und gebunden. Bei der Tinte könnte es sich um eine Flüssigkeit oder ein Trägermittel mit Pigmenten, mit färbenden Ionen/Salzen oder sogar um einen Schlicker handeln.

Danach senkt sich der Wannenboden der zweiten Wanne erneut ab, und es wird eine neue Pulverschicht aufgetragen und dann durch den Drucker ortsselektiv eingefärbt gebunden. Nach dem Drucken und Binden der letzten Schicht wird das den aufgebauten Körper umgebende ungebundene Pulver entfernt, so dass der gewünschte Form-Körper übrig bleibt. Bei den 3-D-Tintenstrahldruckverfahren wird das Baumaterial (Keramik oder Glaspartikel) mit dem selektiv eingefärbten Bindemittel ortsselektiv verdruckt, wobei die Druckköpfe prinzipiell wie Tintenstrahldruckköpfe arbeiten. Ein derartiges 3-D-Tintenstrahldruckverfahren ist z.B. in EP 2 783 837 A1 beschrieben.

Neben der nicht gegebenen Notwendigkeit eines gesonderten Entbinderungs- und Sinterschritts können darüber hinaus einfachere Bindersysteme verwendet werden, da eine Sinterung nicht notwendig ist und somit die Gründichte nicht entscheidend ist.

Ferner ist die Verwendung von sphärischen Glaskugeln, z.B. mit Durchmessern im Bereich von 20 µm bis 100 µm, möglich, wodurch bessere Rieselfähigkeit sowie geringe Binderanteile möglich sind. Durch die sphärische Form der Glaskugeln wird die Dosierbarkeit verbessert. Durch die Größe der Glaskugeln werden die Auflösungsgenauigkeit und die Anforderungen an das Gerät festgelegt. Je größer die Glaskugeln, desto geringer ist Auflösung, d.h. desto geringer ist Detailgenauigkeit des aufgebauten Formkörpers. Neben Glaskugeln ist grundsätzlich jedoch auch die Verwendung von Glaspulver, Granulaten, etc. möglich.

Ferner ermöglicht der an die herzustellende Dentalrestauration individuell angepasste Rohling es auch, dass die Menge an Rohlingsmaterial genauso angepasst wird, dass gerade genug Material für die herzustellende Dentalrestauration zur Verfügung steht, was gegenüber standardisierten Rohlingen Materialeinsparung erlaubt.

Wie oben erwähnt ist es möglich, Rohlinge mit einfachen Geometrien (Kreiszylinder, Quader, Dreieckszylinder oder Zylinder mit sechseckiger Grundfläche etc.) zu verwenden. Es ist jedoch bevorzugt, insbesondere wenn die ortsabhängige Verteilung von Farbe und/oder Opazität und/oder Strukturmerkmalen im Rohling nicht drehsymmetrisch ist, dass die Rohlinge mit einer Verdrehsicherung versehen sind, z.B. einem Vorsprung an einer Stelle ihres Umfangs. Der Pressraum der Pressmuffel ist dann mit einer entsprechenden komplementären Ausnehmung versehen, so dass der Rohling nur in einer vorgegebenen Drehstellung in den Pressraum eingeführt werden kann. Dadurch wird sichergestellt, dass die durch Rücksimulation der in der Dentalrestauration gewünschten Verteilung von Farbe und/oder Opazität gewonnene entsprechende Verteilung der Farbe und/oder Opazität im Rohling auch so positioniert im Pressraum liegt, dass beim Pressen und Fließen des Materials durch die Kanalstruktur der gewünschte Fließprozess hervorgerufen wird, der gerade die gewünschte Verteilung von Farbe und/oder Opazität in der verpressten Dentalrestauration ergibt.

Die Erfindung wird im Folgenden anhand eines Ausführungsbeispiels in den Zeichnungen beschrieben, in denen:
Fig. 1 ein einem Flussdiagramm ähnliches Schaubild zur Veranschaulichung des Ablaufs des erfindungsgemäßen Verfahrens zeigt,
Fig. 2 eine Querschnittsansicht einer im Zusammenhang mit dem erfindungsgemäßen Verfahren verwendbaren Druckvorrichtung zum 3-D-Pulverdruck zeigt,
Fig. 3 eine schematische Querschnittsansicht einer Pressmuffel, in der ein Modell für einen Formhohlraum der Dentalrestauration und für eine Kanalstruktur angeordnet und von einer Einbettungsmasse im Inneren der Pressmuffel umgeben ist,
Fig. 4 bis 6 schematische Querschnittsansichten aufeinanderfolgender Zustände beim Pressen des Rohlings aus einem Pressraum und in die Kanalstruktur und den Formhohlraum hinein zeigen.

Zunächst wird die Herstellung der Pressform kurz erläutert, die für das Pressverfahren benötigt wird, das beim Verfahren der vorliegenden Erfindung angewendet wird. Eine Pressmuffel ist allgemein mit 20 bezeichnet und umfasst eine Pressmuffelhülse 22 und eine Pressmuffelbasis 24, die lösbar mit der Pressmuffelhülse 22 verbunden ist. Auf einem Aufsatz auf der Pressmuffelbasis 24 ist mittig ein Vorsprung 26 befestigt, der in das Innere der Pressmuffelhülse 22 vorragt. Der Vorsprung 26 dient zur Anbringung des Modells der Kanalstruktur 114 und des daran anschließenden Modells 112 der Dentalrestauration. Das Modell 114 für die Kanalstruktur, das sonst auch als Anstiftsystem bezeichnet wird, und das Modell 112 für den Formhohlraum bestehen aus einem vollständig ausbrennbaren Material, zum Beispiel aus Wachs oder aus Kunststoff. Das Modell 112 für die herzustellende Dentalrestauration und die zugehörige Kanalstruktur 114 sind zuvor anhand eines 3-D-Datenmodelles für die herzustellende Dentalrestauration erstellt worden, wobei dies durch aufbauende Verfahren wie 3-D-Pulverdruck oder 3-D-Tintenstrahldruck, durch abtragende Verfahren wie Fräsen oder durch manuelle Anfertigung erfolgen kann. Nach Anbringen des Modells 114 für die Kanalstruktur und des Modells 112 für die Dentalrestauration an dem Vorsprung 26 im Inneren der Pressmuffelhülse 22 wird diese vollständig mit einer Einbettungsmasse gefüllt. Die Einbettungsmasse kann eine gipsähnliche, phosphatgebundene Zusammensetzung haben, mit z.B. Quarzmehl, und ist zunächst fließfähig und härtet nach dem Einfüllen in die Pressmuffel zu der Pressform 28 aus. Nach dem Aushärten kann die Pressmuffelbasis 24 von der Pressmuffelhülse 22 gelöst und mit dem daran befestigten Vorsprung 26 entfernt werden. Nach Ausbrennen der Materialien des Modells 112 für die Dentalrestauration und des Modells 114 für die Kanalstruktur verbleiben dort zu den Modellen komplementäre Hohlräume in der Pressform 28. In dem Bereich des entfernten Vorsprungs 26 verbleibt ein Pressraum, der an dem in der Einbettungsmasse liegenden Ende in Verbindung mit der hohlen Kanalstruktur steht. In den Pressraum kann ein Rohling aus Keramik oder Glas eingeführt werden, der dann mit einem Druckkolben ins Innere des Pressraums gedrückt wird, wodurch Material des Rohlings durch die Kanalstruktur 114 in den Formhohlraum gedrückt und dort verpresst wird, um die Dentalrestauration mit einer Form wie die Form des Modells 112 für die Dentalrestauration zu bilden.

Wie in EP 2 065 012 B1 beschrieben, kann ein Rohling mit Teilbereichen unterschiedlicher Farbe und/oder Opazität zum Pressen eingesetzt werden, wobei durch die Verteilung von Farbe/Opazität im Rohling und die Fließvorgänge durch die Kanalstruktur und in den Formhohlraum die Verteilung von Farbe/Opazität im verpressten Zustand in dem Formhohlraum und damit in der hergestellten Dentalrestauration festgelegt ist. Dabei wurden bei dem in EP 2 065 012 B1 beschriebenen Verfahren aber nur einige Standard-Rohling mit gewissen Variationen der Verteilungen von Farbe/Opazität verwendet und eine gewisse Variation hin zu dem gewünschten Ergebnis durch Anpassung der Geometrie der Kanalstruktur (zur Beeinflussung der Fließvorgänge) bewirkt. Im Zusammenhang mit der vorliegenden Erfindung ist eine individuelle Anpassung der Kanalstruktur nicht erforderlich.

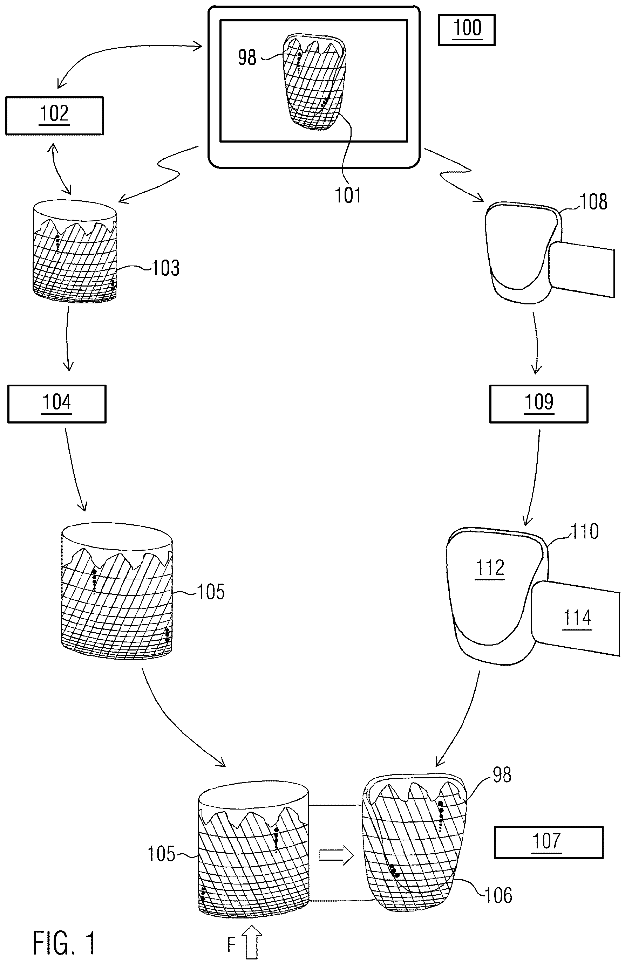

Das Verfahren nach der vorliegenden Erfindung wird nun zunächst unter Bezugnahme auf Figur 1 erläutert. Mithilfe einer Datenverarbeitungseinheit 100 wird in bekannter Weise ein 3-D-Datenmodell 101 der gewünschten Dentalrestauration erzeugt, das in Fig. 1 auf einer neben der Datenverarbeitungseinrichtung 100 angeordneten Anzeige dargestellt ist. Das 3-D-Datenmodell 101 der Dentalrestauration ist mit Gitterlinien versehen, deren Dichte über das Volumen der Dentalrestauration variiert. Dadurch soll schematisch eine ortsabhängige Verteilung von Farbe und/oder Opazität im Volumen der Dentalrestauration dargestellt sein. Oben sind an dem 3-D-Datenmodell 101 Mamelons an der Inzisalkante angedeutet. Ferner enthält das 3-D-Datenmodell 101 weitere Strukturmerkmale, wie Schmelzflecken 98. Nach der vollständigen Erstellung des 3-D-Datenmodells der Dentalrestauration wird in Schritt 102 ein Prozess einer umkehrenden Abbildung von dem 3-D-Datenmodell 101 der Dentalrestauration in ein 3-D-Datenmodell 103 des Rohlings durchgeführt, der bei Berücksichtigung der Fließvorgänge beim Pressen des Rohlings gemäß diesem 3-D-Datenmodell in der Kanalstruktur und in den Formhohlraum eine Verteilung von Farbe und/oder Opazität und Strukturmerkmalen in der erzeugten Dentalrestauration ergibt, die mit dem 3-D-Datenmodell 101 der gewünschten Dentalrestauration übereinstimmt. Eine solche umkehrende Abbildung von dem gewünschten Produkt mit dem Aufbau des 3-D-Datenmodells 101 der Dentalrestauration auf die entsprechende Verteilung von Farbe und/oder Opazität und Strukturmerkmalen in das 3-D-Datenmodell 103 des Rohlings, der als Eingangsprodukt in den Pressvorgang eingesetzt werden soll, kann durch Simulation der Fließvorgänge des Materials durch die Kanalstruktur und in den Formhohlraum hinein bis zu dessen vollständige Auffüllung erhalten werden, indem z.B. die Verteilung in dem 3-D-Datenmodell 103 des Rohlings so variiert wird, bis die nach Simulation des Pressvorgangs resultierende Verteilung von Farbe und/oder Opazität und Strukturmerkmale in dem simulierten Ergebnis des Pressvorgangs mit dem 3-D-Datenmodell 101 der gewünschten Dentalrestauration übereinstimmt. Derartige Simulationen von Fließvorgängen und entsprechende Variation der Ausgangsverteilung von Farbe und/oder Opazität, so dass sich eine gewünschte Verteilung nach dem Fließvorgang ergibt, sind dem Fachmann bekannt. Im Ergebnis ist dies eine umkehrende Abbildung durch umgekehrte Fließsimulation aus dem gewünschten 3-D-Datenmodell der Dentalrestauration in ein entsprechendes 3-D-Datenmodell 103 des Rohlings.

Der dem 3-D-Datenmodell 103 des Rohlings entsprechende physische Rohling 105 mit der gewünschten Verteilung von Farbe und/oder Opazität und/oder Strukturmerkmalen wird dann in Schritt 104 durch ein schichtweise aufbauendes, generatives Bauverfahren aufgebaut, um den physischen Rohling 105 zu erhalten.

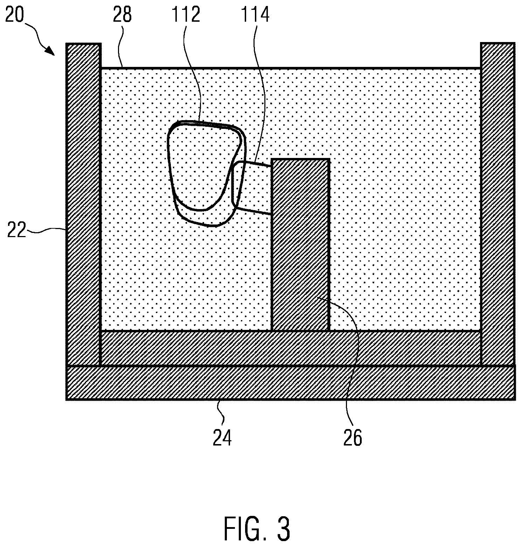

Neben der Herstellung des individualisierten Rohlings 105 ist in dem rechten Zweig des Prozesses ausgehend von der Datenverarbeitungseinrichtung 100 die Herstellung eines Modells dargestellt, mit dem nach dem Prinzip der verlorenen Form eine Einpressform für den Pressvorgang für die Dentalrestauration erstellt werden soll. Zunächst wird ein 3-D-Datenmodell 108 für die Form des Formhohlraums für die Dentalrestauration und die darin anschließende Kanalstruktur generiert. In Schritt 109 wird dann aus dem 3-D-Datenmodell 108 in einem Herstellungsverfahren ein entsprechendes physisches Modell 110 mit einem physischen Modell 112 für die Dentalrestauration und einem daran anschließenden Modell 114 für die Kanalstruktur erzeugt. Dieses Modell 110 wird aus einem vollständig ausbrennbarem Material wie etwa Wachs oder Plexiglas hergestellt. Das Herstellungsverfahren im Schritt 109 unterliegt dabei keinen Beschränkungen, es können zum Beispiel CAM-Verfahren zur Anwendung kommen, die aus dem 3-D-Datenmodell 108 durch generativ aufbauende Verfahren oder durch abtragende Verfahren (Fräsen) das physische Modell 110 erzeugten, das dann wie im Zusammenhang mit Fig. 3 erläutert in das Innere der Pressmuffelhülse 22 eingebracht und dort von Einbettungsmasse umgossen wird, um schließlich die ausgehärtete Einpressform 28 zu erhalten, die dann nach Ausbrennen des Modells 110 die gewünschte Hohlraumstruktur hat. In den durch den Vorsprung 26 in der Pressform 28 erzeugten Pressraum wird dann der individualisierte Rohling 105 eingesetzt. Durch Einpressen des individualisierten Rohlings 105 in den Pressraum wird Material des Rohlings durch die an den Pressraum anschließende Kanalstruktur und in den Formhohlraum der Dentalrestauration in der Pressform gedrückt. Dieser Vorgang ist in Fig. 1 unten im Schritt 107 nur schematisch dargestellt. Da der physische Rohling 105 in dem erfindungsgemäßen Verfahren wie oben beschrieben individualisiert angepasst an die herzustellende Dentalrestauration und der darin gewünschten Verteilung von Farbe und/oder Opazität und/oder Strukturmerkmalen unter umkehrender Abbildung des Pressvorgangs individualisiert erstellt worden ist, ergibt sich nach dem Pressvorgang die physische Dentalrestauration 106 mit der Form und Verteilung von Farbe und/oder Opazität und/oder Strukturmerkmalen wie sie durch das 3-D-Datenmodell 101 für die Dentalrestauration vorgegeben ist, d.h. die physische Dentalrestauration 106 hat die räumliche Verteilung von Farbe, Opazität und Strukturmerkmalen wie durch das 3-D-Datenmodell 101 vorgegeben (die physische Dentalprothese 106 ist in Fig. 1 gegenüber dem 3-D-Datenmodell der Dentalprothese von der anderen Seite gezeigt).

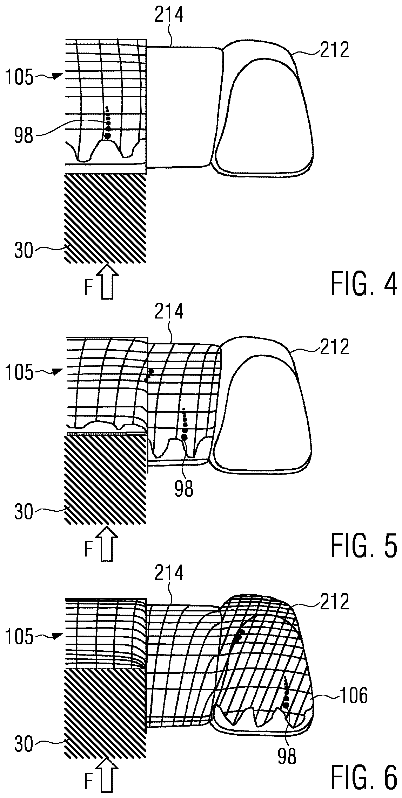

Der Pressvorgang ist in der Folge der Figuren 4 bis 6 etwas detaillierter dargestellt. In Fig. 4 befindet sich der Rohling 105 in dem Pressraum der Pressform (nicht dargestellt). Die Kanalstruktur 214 ist in dem in Fig. 4 dargestellten Ausgangszustand, in dem sich der Rohling 105 im Pressraum befindet, noch vollständig leer. Nun wird mit einem Druckkolben 30 Kraft auf den Rohling 105 ausgeübt, die ihn in den Pressraum hineindrückt. Da die Kanalstruktur 214 in diesem Fall den einzigen Raum bildet, in dem Material ausweichen kann wird durch den Pressvorgang, dem vorzugsweise eine Erhitzung des Rohlings 105 vorhergeht, Material des Rohlings 105 in die Kanalstruktur 214 gedrückt, wie in Fig. 5 dargestellt ist. Nach weiterem Pressen durch den Kolben 30 ist schließlich der Formhohlraum 212 für die Dentalrestauration vollständig aufgefüllt und das Material der Dentalrestauration darin verpresst. In dem dargestellten Ausführungsbeispiel schließt an den Presseraum nur eine Kanalstruktur und ein Formhohlraum an. Grundsätzlich können auch zwei oder mehr Kanalstrukturen von dem Presseraum ausgehen und jeweils zu einem zugehörigen Formhohlraum führen, wenn gleichzeitig mehrere Dentalrestaurationen parallel gepresst werden sollen. Im Falle von zwei oder mehr Kanalstrukturen mit zugehörigen Formhohlräumen müssen diese natürlich alle bei der Simulation der Fließvorgänge und bei der umkehrenden Abbildung von den zu erzielenden Dentalrestaurationen auf einen diese erzeugenden Rohling berücksichtigt werden.

Wie in den Figuren 4 bis 6 durch die Verzerrung der Gitterlinien und durch die Wanderung der Strukturmerkmale 98 angedeutet, wird die durch die Gitterlinien im Rohling 105 angedeutete Verteilung von Farbe und/oder Opazität und die Lage von Feinstrukturen 98 beim Pressvorgang in eine veränderte Gitterstruktur und eine veränderte Lage der Feinstrukturen 98 in der Dentalrestauration 106 transformiert. Nach der vorliegenden Erfindung ist der Rohling 105 mit seiner Verteilung von Farbe und/oder Opazität und/oder Strukturmerkmalen individuell gerade so aufgebaut, dass sich nach der Transformation durch den Pressvorgang gerade Dentalrestauration 16 mit einer Verteilung von Farbe und/oder Opazität und/oder Strukturmerkmalen 98 ergibt, wie sie durch das zugrundeliegende 3-D-Datenmodell 101 der Dentalrestauration vorgegeben ist. Mit anderen Worten wurde bei der in Fig. 1 schematisch im Schritt 102 angedeuteten umkehrenden Abbildung durch Simulation eine Transformation vorgenommen, die ein 3-D-Datenmodell der gewünschten Prothese aus dem Zustand in Fig. 6 durch Umkehrung der Fließprozesse beim Pressvorgang in ein entsprechendes 3-D-Datenmodell für den Rohling 105 im Zustand von Fig. 4 überführt.

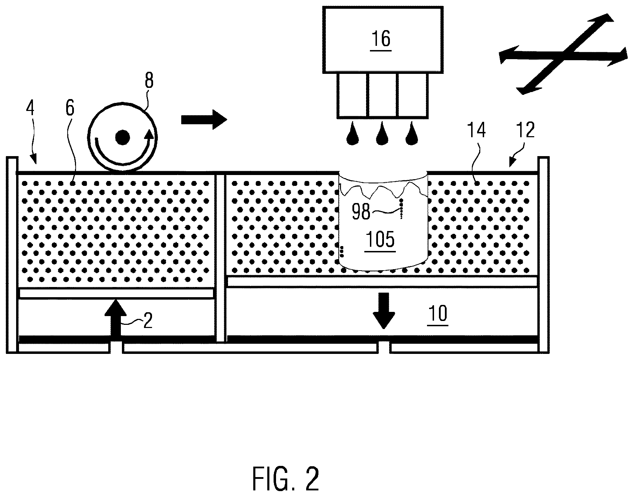

Zur Illustration des individualisierten Aufbauvorgangs für den individualisierten Rohling 105 aus dem 3-D-Datenmodell 103 für den Rohling wird beispielhaft auf die Illustration eines 3-D-Pulverdruckverfahrens in Fig. 2 Bezug genommen. Fig. 2 zeigt eine erste Wanne, die Pulver 6 enthält. Mit einer Transportwalze 8 wird Pulver 6 in eine daneben liegende zweite Wanne 12 und in Form einer Schicht auf einen dort aufzubauenden Rohling 105 transportiert. Nach jedem Auftrag einer neuen Pulverschicht wird ein Tintenstrahldruckkopf 16 in X- und Y-Richtung gesteuert über die Oberfläche des Baubereichs verfahren, wodurch die aufgetragene Pulverschicht mit Bindemittel mit ortsabhängig vorgegebenen Farben oder absorbierenden Substanzen zur Erzeugung von lichtundurchlässigeren Bereichen bedruckt und gebunden wird. Es können auch Bindemittel und Farben separat voneinander aufgedruckt werden. Nach Bedrucken einer Schicht wird die Hubeinrichtung 10 unter der zweiten Wanne 12 um eine Strecke entsprechend einer Schichthöhe abgesenkt, die erste Wanne 4 durch eine erste Hubeinrichtung 2 entsprechend angehoben und mit der Transportwalze 8 wieder frisches Pulver 6 auf die zuletzt gebildete Schicht auf dem Rohling 105 aufgetragen. Dieser Vorgang wiederholt sich, bis der gewünschte Rohling 105 vollständig fertiggesellt ist. Danach wird nicht mit Bindemittel bedrucktes verbleibendes Pulver 14 entfernt und rückgeführt, so dass der gewünschte individualisiert aufgebaute Rohling 105 für das weitere Verfahren zur Verfügung steht.

## Patentansprüche

1. Verfahren zur Herstellung einer Dentalrestauration, bei dem ein Rohling aus Keramik, insbesondere Glaskeramik, oder Glas bereitgestellt wird, der eine vorgegebene Ortsabhängigkeit der Materialeigenschaft Farbe und/oder Opazität in seinem Volumen aufweist, und der Rohling (105) in einen Pressraum einer Pressmuffel (20) gepresst und dadurch Material des Rohlings durch eine von dem Pressraum ausgehende Kanalstruktur in einen der Dentalrestauration entsprechenden Formhohlraum (212) gepresst wird, wobei die Kanalstruktur (214) und die Ortsabhängigkeit von Farbe und/oder Opazität im Rohling (105) aufeinander abgestimmt sind, so dass sich eine aus einer Mehrzahl von möglichen Verteilungen von Farbe und/oder Opazität im keramischen Material der im Formhohlraum (212) gebildeten Dentalrestauration (106) ergibt, **dadurch gekennzeichnet, dass**
aus einer in der herzustellenden Dentalrestauration gewünschten Verteilung von Farbe und/oder Opazität und/oder von optischen Strukturmerkmalen unter Berücksichtigung der Fließwege in der Kanalstruktur die zugrundeliegende Ortsabhängigkeit von Farbe und/oder Opazität und/oder von optischen Strukturmerkmalen (98) im Rohling (105) bestimmt wird, indem
(i) entweder die in der herzustellenden Dentalrestauration gewünschte Verteilung von Farbe und/oder Opazität und von optischen Strukturmerkmalen einer zeitlich rückwärts verlaufenden Simulation der Fließvorgänge beim Pressen des Materials durch die Kanalstruktur (214) in den Formhohlraum (212) unterzogen wird, um so diejenige Ortsabhängigkeit von Farbe und/oder Opazität im Rohling zu erhalten, die nach Pressen des Materials in den Formhohlraum (212) dort die gewünschte Verteilung von Farbe und/oder Opazität und/oder von optischen Strukturmerkmalen (98) ergibt,
(ii) oder eine vorgegebene Ortsabhängigkeit von Farbe und/oder Opazität und/oder von optischen Strukturmerkmalen (98) im Rohling (105) einer zeitlich vorwärts verlaufenden Simulation der Fließvorgänge beim Pressen des Materials durch die Kanalstruktur (214) in den Formhohlraum (212) unterzogen wird, um so die jeweils resultierende Ortsabhängigkeit von Farbe und/oder Opazität und/oder Strukturmerkmalen im Formhohlraum (212) zu erhalten, und die genannte vorgegebene Ortsabhängigkeit im Rohling variiert und die zeitlich vorwärts verlaufende Simulation wiederholt wird, bis die resultierende Ortsabhängigkeit von Farbe und/oder Opazität und/oder Strukturmerkmalen im Formhohlraum (212) mit der gewünschten Verteilung von Farbe und/oder Opazität und/oder von Strukturmerkmalen (98) in der Dentalrestauration übereinstimmt,
der Rohling (105) bereitgestellt wird, indem er in einem aufbauenden Verfahren mit der bestimmten Ortsabhängigkeit von Farbe und/oder Opazität und/oder von optischen Strukturmerkmalen (98) individuell aufgebaut wird.

2. Verfahren nach Anspruch 1, **dadurch gekennzeichnet, dass** der Rohling vor und oder beim Pressen erhitzt wird.

3. Verfahren nach einem der vorhergehenden Ansprüche, **dadurch gekennzeichnet, dass** der Rohling (105) nach seinem Aufbau ohne weitere Zwischenschritte direkt in den Pressraum der Pressmuffel eingesetzt wird, um eine Dentalrestauration (106) herzustellen.

4. Verfahren nach einem der Ansprüche 1 oder 2, **dadurch gekennzeichnet, dass** der aufgebaute Rohling (105) vor Einbringung in den Presseraum vorgesintert oder dichtgesintert wird.

5. Verfahren nach einem der vorhergehenden Ansprüche, **dadurch gekennzeichnet, dass** der Rohling (105) durch ein Stereolithographie-, 3-D-Pulverdruck- oder 3-D-Tintenstrahldruckverfahren aufgebaut wird.

6. Verfahren nach einem der vorhergehenden Ansprüche, **dadurch gekennzeichnet, dass** für den Aufbau des Rohlings (105) Glas mit Keimen verwendet wird.

7. Verfahren nach einem der einem der Ansprüche 1 bis 4, **dadurch gekennzeichnet, dass** für den Aufbau des Rohlings (105) eine Glaskeramik mit der Hauptkristallphase Lithium-Metasilicat, Lithium-Disilicat oder SiO₂-Phasen oder Zwischenstufen davon verwendet wird.

## Claims

1. A method for producing a dental restoration, in which method a blank is made available which is made from ceramic, in particular glass ceramic, or glass and which has a predefined spatial dependency of the material property color and/or opacity in its volume, and the blank (105) is pressed into a press chamber of a press muffle (20) and, in this way, material of the blank is pressed, through a channel structure issuing from the press chamber, into a mold cavity (212) corresponding to the dental restoration, wherein the channel structure (214) and the spatial dependency of color and/or opacity in the blank (105) are coordinated with each other, such that one of a plurality of possible distributions of color and/or opacity is obtained in the ceramic material of the dental restoration (106) formed in the mold cavity (212), **characterized in that**
the basic spatial dependency of color and/or opacity and/or of optical structural features (98) in the blank (105) is determined from a desired distribution of color and/or opacity and/or of optical structural features in the dental restoration to be produced, taking account of the flow paths in the channel structure, wherein this determination is carried out
i) either by subjecting the desired distribution of color and/or opacity and of optical structural features in the dental restoration to be produced to a chronologically backwards simulation of the flow processes during the pressing of the material through the channel structure (214) into the mold cavity (212), in order thereby to obtain, in the blank, the spatially dependency of color and/or opacity which, after the material being pressed into the mold cavity (212), provides there the desired distribution of color and/or opacity and/or of optical structural features (98),
ii) or by subjecting a predetermined spatial dependency of color and/or opacity and/or of optical structural (98) features in the blank (105) to a chronologically forwardly directed simulation of the flow processes during the pressing of the material through the channel structure into the mold cavity (212), to thereby obtain the resulting spatial dependency of color and/or opacity and/or of optical structural features in the mold cavity (212), and by varying said predetermined spatial dependency of color and/or opacity and/or of optical structural features and repeating said chronologically forwardly directed simulation until the resulting spatial dependency of color and/or opacity and/or of optical structural features in the mold cavity (212) coincides with desired spatial dependency of color and/or opacity and/or of optical structural features (98) in the dental restoration,
and
the blank (105) is made available by being built up individually in an additive method with the determined spatial dependency of color and/or opacity and/or of optical structural features (98).

2. The method as claimed in claim 1, **characterized in that** the blank is heated before and/or during the pressing.

3. The method as claimed in any of the preceding claims, **characterized in that** the blank (105), after it has been built up, is inserted without any further intermediate steps directly into the press chamber of the press muffle, in order to produce a dental restoration (106).

4. The method as claimed in any of claims 1 and 2, **characterized in that** the built-up blank (105) is pre-sintered or dense-sintered before being introduced into the press chamber.

5. The method as claimed in any of the preceding claims, **characterized in that** the blank (105) is built up by a stereolithography, 3D powder printing or 3D inkjet printing method.

6. The method as claimed in any of the preceding claims, **characterized in that** glass with seeds is used for building up the blank (105).

7. The method as claimed in one of one of claims 1 through 4, **characterized in that** the blank (105) is built up using a glass ceramic with the main crystal phase lithium metasilicate, lithium disilicate or SiO₂ phases or intermediates thereof.

## Revendications

1. Procédé pour la fabrication d'une restauration dentaire, où une ébauche en céramique, en particulier en vitrocéramique ou en verre, est fournie, qui présente une dépendance locale prédéfinie de la propriété de matériau couleur et/ou opacité dans son volume, et l'ébauche (105) est pressée dans un espace de pressage d'un moufle de pressage (20) et ainsi la matière de l'ébauche est pressée à travers une structure de canaux partant de l'espace de pressage dans un espace creux de moulage (212) correspondant à la restauration dentaire, dans lequel la structure de canaux (214) et la dépendance locale de couleur et/ou d'opacité dans l'ébauche (105) sont adaptées l'une à l'autre de sorte qu'il en résulte une parmi une multitude de répartitions possibles de couleur et/ou d'opacité dans la matière céramique de la restauration dentaire (106) formée dans l'espace creux de moulage (212), **caractérisé en ce que**
est définie, à partir d'une répartition, souhaitée dans la restauration dentaire à fabriquer, de couleur et/ou d'opacité et/ou de caractéristiques structurales optiques, tout en tenant compte des voies d'écoulement dans la structure de canaux, la dépendance locale de base de couleur et/ou d'opacité et/ou de caractéristiques structurales (98) optiques dans l'ébauche (105) **en ce que**
- (i) soit la répartition, souhaitée dans la restauration dentaire à fabriquer, de couleur et/ou d'opacité et de caractéristiques structurales optiques est soumise à une simulation rétroactive des opérations d'écoulement lors du pressage de la matière à travers la structure de canaux (214) dans l'espace creux de moulage (212) pour obtenir ainsi la dépendance locale de couleur et/ou d'opacité dans l'ébauche qui précisément donne lieu à cet endroit, après le pressage de la matière dans l'espace creux de moulage (212), à la répartition souhaitée de couleur et/ou d'opacité et/ou de caractéristiques structurales (98) optiques,
- (ii) soit une dépendance locale prédéfinie de couleur et/ou d'opacité et/ou de caractéristiques structurales (98) optiques dans l'ébauche (105) est soumise à une simulation proactive des opérations d'écoulement lors du pressage de la matière à travers la structure de canaux (214) dans l'espace creux de moulage (212) pour obtenir ainsi la dépendance locale résultante respectivement de couleur et/ou d'opacité et/ou de caractéristiques structurales dans l'espace creux de moulage (212), et ladite dépendance locale prédéfinie varie dans l'ébauche et la simulation proactive est répétée jusqu'à ce que la dépendance locale résultante de couleur et/ou d'opacité et/ou de caractéristiques structurales dans l'espace creux de moulage (212) coïncide avec la répartition souhaitée de couleur et/ou d'opacité et/ou de caractéristiques structurales (98) dans la restauration dentaire,
l'ébauche (105) est fournie **en ce qu'**elle est produite individuellement dans un procédé de production avec la dépendance locale définie de couleur et/ou d'opacité et/ou de caractéristiques structurales (98) optiques.

2. Procédé selon la revendication 1, **caractérisé en ce que** l'ébauche est chauffée avant ou lors du pressage.

3. Procédé selon l'une quelconque des revendications précédentes, **caractérisé en ce que** l'ébauche (105) est insérée, après sa production, sans étapes intermédiaires supplémentaires, directement dans l'espace de pressage du moufle de pressage pour fabriquer une restauration dentaire (106).

4. Procédé selon l'une quelconque des revendications 1 ou 2, **caractérisé en ce que** l'ébauche (105) produite est frittée au préalable ou est frittée à densité maximale avant l'introduction dans l'espace de pressage.

5. Procédé selon l'une quelconque des revendications précédentes, **caractérisé en ce que** l'ébauche (105) est produite par un procédé d'impression par stéréolithographie, un procédé d'impression à poudre 3D ou un procédé d'impression à jet d'encre 3D.

6. Procédé selon l'une quelconque des revendications précédentes, **caractérisé en ce que** du verre avec des germes est utilisé pour la production de l'ébauche (105).

7. Procédé selon l'une quelconque des revendications 1 à 4, **caractérisé en ce qu'**est utilisée pour la production de l'ébauche (105) une vitrocéramique avec la phase cristalline principale métasilicate de lithium, disilicate de lithium ou des phases de SiO₂ ou des étapes intermédiaires de celles-ci.
